# EUROPEAN PATENT SPECIFICATION

(11) **EP 2 770 922 B1**
(45) Date of publication and mention of the grant of the patent: **12.04.2017**
(21) Application number: 12791273.1
(22) Date of filing: 30.10.2012
(51) Int. Cl.: A61B 17/34

(54) **AN INSTRUMENT ACCESS DEVICE**
INSTRUMENTENZUGRIFFSVORRICHTUNG
DISPOSITIF D'ACCÈS D'INSTRUMENT

(30) Priority: 28.10.2011 US 201161552851 P
(43) Date of publication of application: 03.09.2014
(73) Proprietor: Atropos Limited, Bray, County Wicklow (IE)
(72) Inventor: BONADIO, Frank, Bray County Wicklow (IE); VAUGH, Trevor, Birr County Offaly (IE)
(74) Representative: O'Brien, John Augustine
(86) International application number: PCT/IE2012/000046
(87) International publication number: WO 2013/061314

(56) References cited:
- EP-A2- 2 238 932
- IE-A1- 20 050 688
- US-A1- 2005 165 281
- US-A1- 2005 192 483
- US-A1- 2011 028 793

## Description

### Introduction

The invention relates to an instrument access device and an instrument access system incorporating the instrument access device.

IE2005688 A describes an instrument access device comprising a distal ring for insertion into a wound interior, a proximal member for location externally of a wound opening and a sleeve extending in two layers between the distal O-ring and the proximal member. The proximal member comprises an inner proximal ring member and an outer proximal ring member between which the sleeve is led. A seal housing is mounted to the inner proximal ring member. A gelatinous elastomeric seal with a pinhole opening therethrough is received in the housing. An instrument may be extended through the seal to access the wound interior through the retraced wound opening in a sealed manner.

EP2238932A describes a surgical access device having removable and replaceable components.

US2005/192483A describes an instrument access port comprising a retractor for retracting the sides of an incision and a valve for sealing around an instrument inserted through the retracted incision. The retractor comprises a distal member for insertion into the incision, a proximal member for location externally of the incision and a retracting member for extending between the distal member and the proximal member. The valve is coupled to the retractor to define a low profile sealed instrument access port.

### Statements of Invention

According to the invention there is provided an instrument access device as defined in claim 1.

In the invention the cannula has a distal end which extends in use distally of the distal ring of the retractor. However, the distal end of the cannula is located adjacent to and as close as possible to the distal ring to provide minimum obstruction to movement of an instrument within the abdomen.

In one embodiment the inner proximal ring engagement element comprises a receiving slot.

The invention also provides an instrument receiver having a cannula portion which is at least partially flexible.

The length of the cannula portion may be adjustable.

In one case at least portion of the cannula portion is of a material which can be severed to shorten the length of the cannula portion. There may be length-indicating indicia on the cannula.

In one embodiment the cannula is provided with at least one external projection. The projection may be in the form of a spiral or screw thread to aid hoisting motion as the cannula is inserted. The outer surface of the cannula may have ridges and/or corrugations to provide extra grip.

In one case there is a releasable engagement between the cannula and the proximal assembly of the retractor. The releasable engagement may be provided by a panel on the proximal assembly which is engagable with a receiver such as a grove on the cannula.

The valve assembly may comprise a first valve and a second valve distal of the first valve.

In one case the first valve comprises a lip-seal valve.

The second valve may comprise at least two cusps such as a duckbill valve.

In one case the lipseal is provided in a lipseal housing and the second valve is provided in a second seal housing. The lipseal housing may be movable relative to the second seal housing. The lip seal housing may comprise a cap for the second seal housing. In one case the lipseal housing is removable from the second seal housing. The lipseal housing may be releasably connected to the second seal housing. The lipseal housing may be connected to the second seal housing by a hinge connection such as a strap. The lipseal housing may comprise a reducer cap.

In one embodiment the device comprises a distal anchoring member for location within a wound interior; and a retractor member extending proximally from the distal anchoring member to retract laterally the sides of a wound opening.

The retractor member may extend at least between the distal anchoring member and the proximal member. The retractor member may extend in two layers between the distal anchoring member and the proximal member.

In one case a first end portion of the retractor member is fixed to the proximal member.

The retractor member may be movable relative to the distal anchoring member. A second end portion of the retractor member may be movable relative to the proximal member.

In one embodiment the retractor member extends distally from the proximal member to the distal anchoring member, is looped around the distal anchoring member, and extends proximally from the distal anchoring member to the proximal member.

The proximal member may comprise an inner part and an outer part. The retractor member may extend between the inner part and the outer part.

It will be appreciated that features described with reference to one embodiment of the invention may be utilised with any of the other embodiments.

### Brief Description of the Drawings

The invention will be more clearly understood from the following description of some embodiments thereof, given by way of example only, with reference to the accompanying drawings, in which: -
Fig. 1 is an exploded isometric view of an instrument access device according to the invention;
Fig. 2 is a cross sectional view of the assembled device;
Fig. 3 is a cross sectional view of base portion of the device;
Fig. 4 is another isometric view of the device;
Fig. 5 is an elevational view of the device of Fig. 4;
Figs. 6 and 7 are enlarged isometric views illustrating one method of mounting a trocar portion to a base portion of the device;
Fig. 8 is an isometric view from one side of an introducer according to the invention;
Fig. 9 is an isometric view from an opposite side of the introducer;
Figs. 10 and 11 are enlarged views of a distal end of the introducer;
Figs. 12 to 15 are isometric views illustrating various steps using the introducer;
Fig. 16 is an enlarged cross sectional view of the introducer with a distal ring of a wound protector in place;

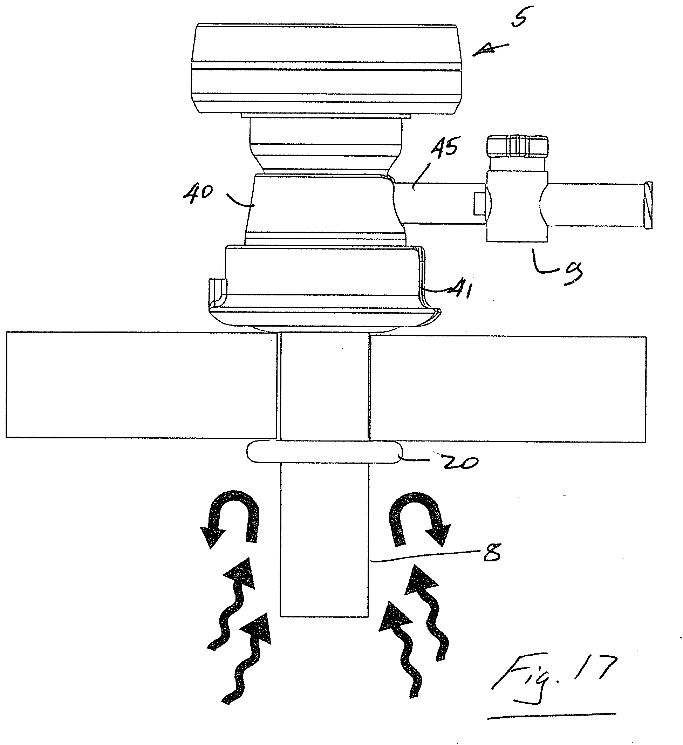

Fig. 17 is a view of the instrument access device in use;
Figs. 18 and 19 are diagrams illustrating the device, in use;
Fig. 20 us an exploded isometric view of another instrument access device according to the invention;
Fig. 21 is a cross sectional view of the assembled device of Fig. 20;
Fig. 22 is an exploded view of another instrument access device;
Fig. 23 is an exploded view of a further instrument access device;
Fig. 24 is an exploded view of another instrument access device;
Fig. 25 is a view of part of the device of Fig. 22 assembled;
Fig. 26 is an isometric view of another instrument access device;
Figs. 27 and 28 are cross sectional views illustrating the device of Fig. 26, in use;
Fig. 29 is a view of another instrument access device according to the invention;
Fig. 30 is a cross sectional view of the device of Fig. 29, in use;
Fig. 31 is a cross sectional view of two of the devices of Fig. 29, in use; and
Fig. 32 is a cross sectional view of another instrument access device according to the invention, in use.

### Detailed Description

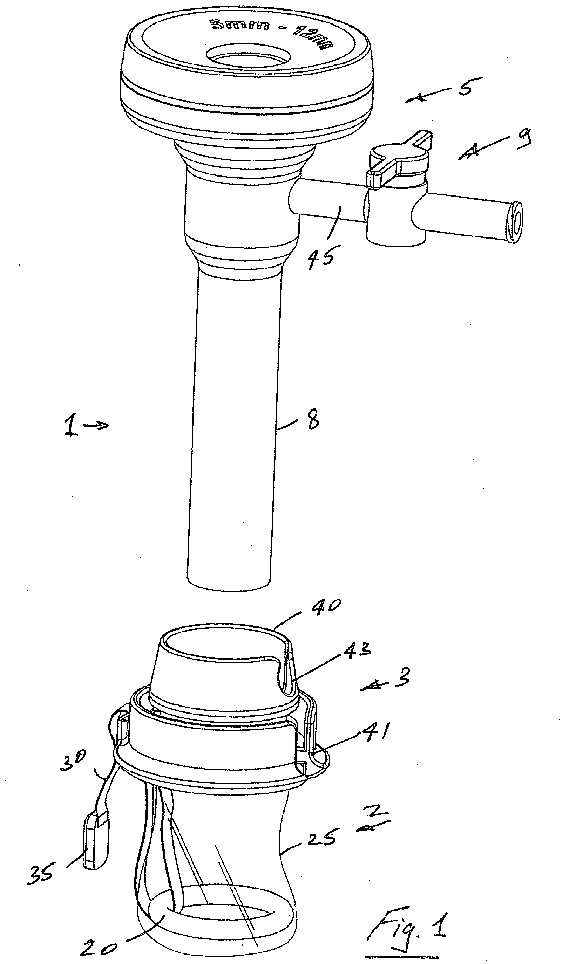

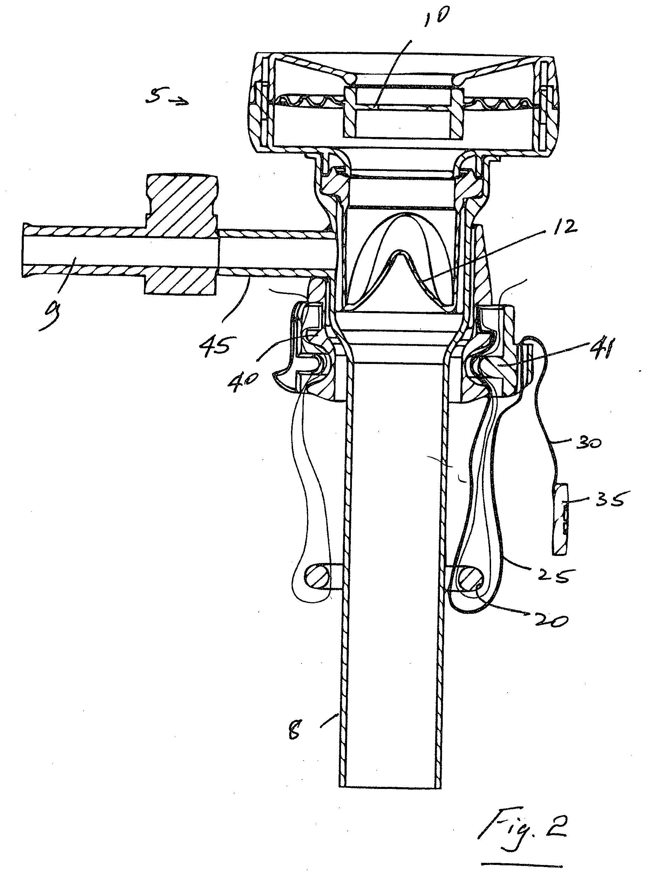

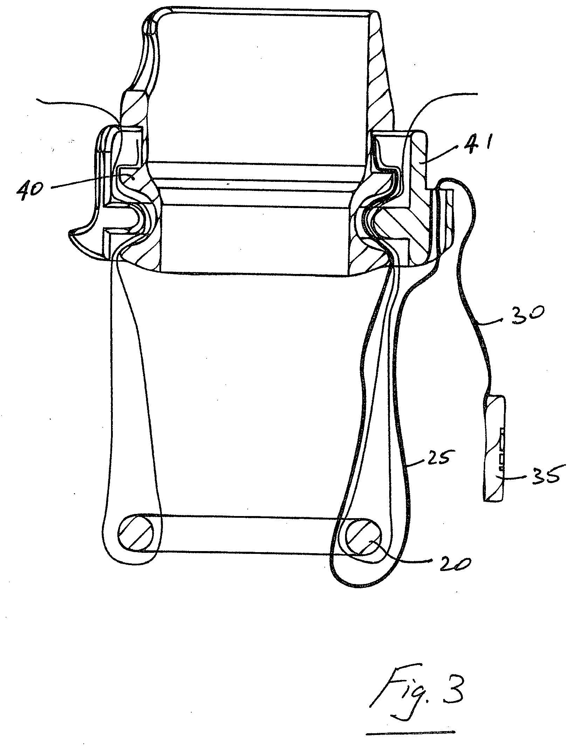

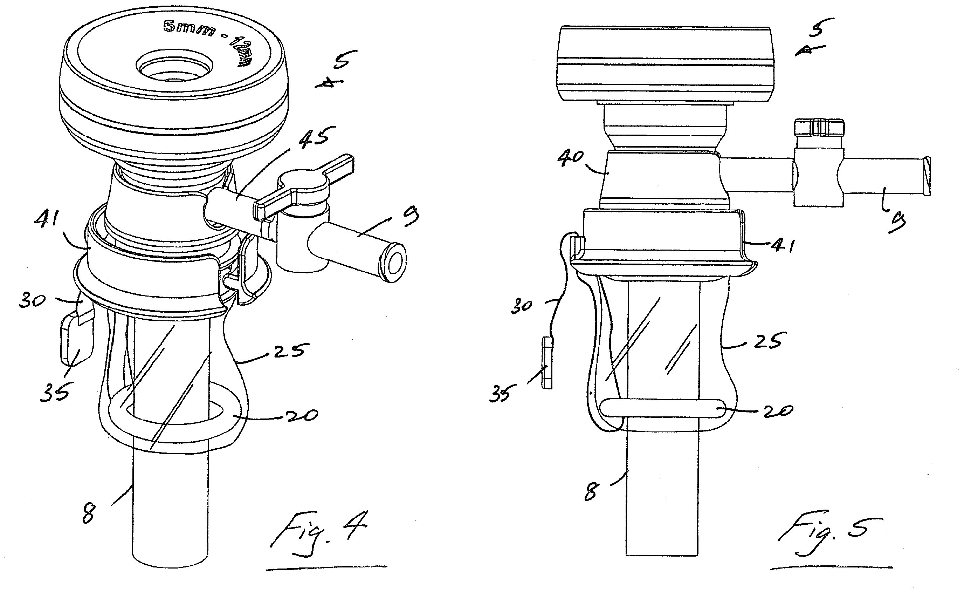

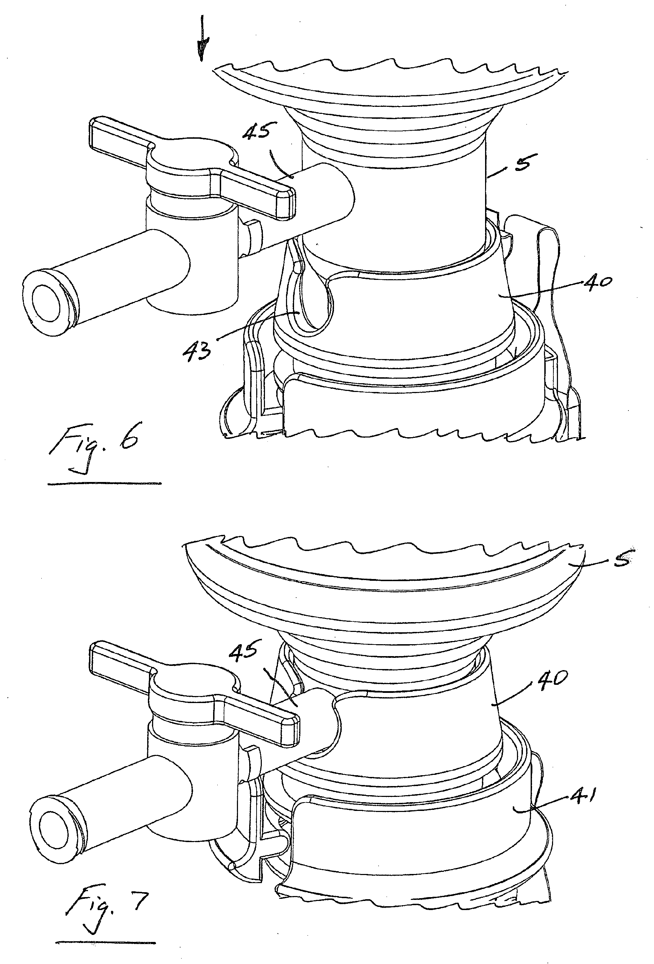

Referring to the drawings and initially to Figs. 1 to 7 thereof, there is illustrated an instrument access device 1 comprising a wound protector and retractor 2, a proximal assembly 3 coupled to the wound protector and retractor 2, and an instrument receiver 5 which can be releasably coupled to the proximal assembly 3. The instrument receiver 5 has a valve assembly 6 through which an instrument 7 is passed and a cannula portion 8 which extends in use through an incision which has been retracted and protected by the retractor 2. The instrument receiver 5 also has an insufflation/desufflation port 9.

A cannula in this context also may be termed an instrument working channel.

In this case the wound retractor device 2 has a distal retaining ring 20 for location distally of the wound opening to retain the wound retractor device 2 in position retracting the incision. For example, the wound retractor device may be of the type described in US6,846,287 and/or US6,582,364, and/or US7,559,893. The retractor includes a sleeve 25 which in this case extends in two layers between the distal anchoring ring 20 and the proximal ring assembly 3. A ribbon 30 attached to a pull ring 35 may be used to aid release of the retractor.

In use, a wound opening is created in a tissue wall and the distal anchoring ring 20 is inserted through an incision opening into the wound interior. The proximal ring assembly 3 is located externally of the incision, with the retractor sleeve 25 extending proximally from the distal anchoring member 20 through the wound opening. The second end of the retractor sleeve 25 is pulled proximally relative to the proximal ring assembly 3 to retract laterally the sides of the wound opening. The instrument receiver 5 is mounted to the proximal ring assembly 3. An instrument 8 may then be inserted through the instrument receiver 5 and extended through the retracted wound opening and into the wound interior.

The proximal assembly 3 comprises an inner proximal ring member 40 and an outer proximal ring member 41. The proximal end of the retractor sleeve 25 is located between the proximal ring members 40, 41. In this case the outer proximal ring member 41 is c-shaped for ease of assembly. The inner proximal ring member 40 in this case has a slot 43 for receiving and releasably engaging with an insufflation port section 45 of the instrument receiver 5.

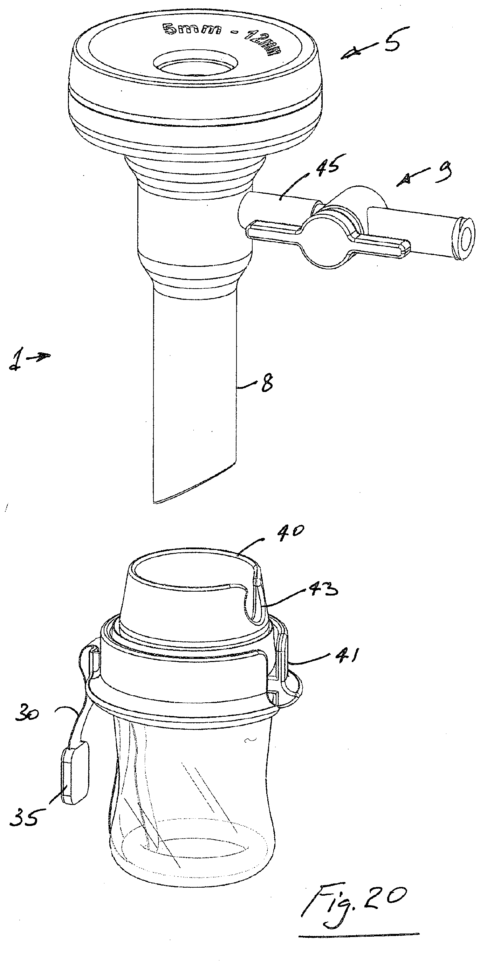

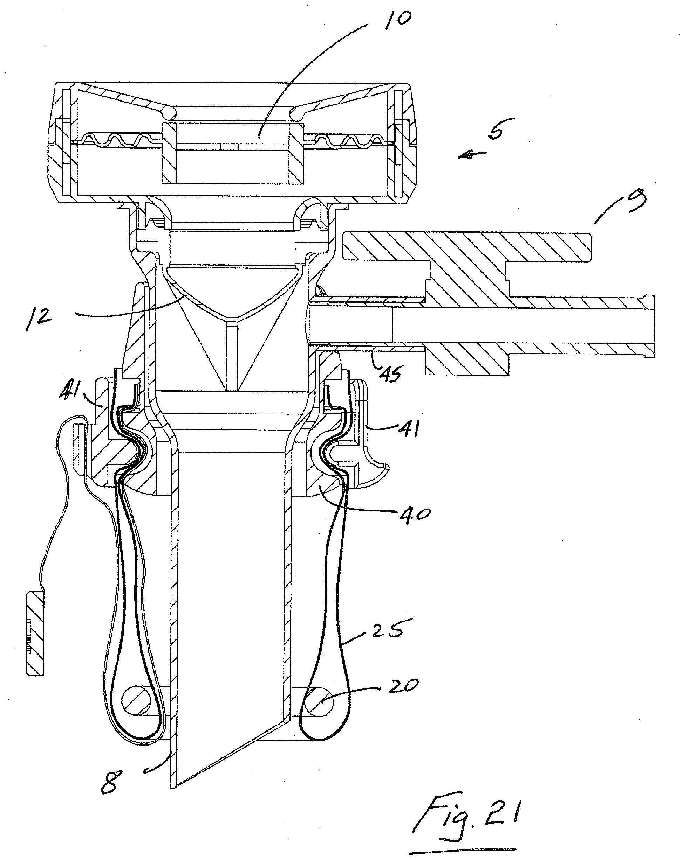

Because the cannula portion 8 extends through the retracted and protected incision there is minimal frictional resistance to the insertion of an instrument through the retracted incision. In some cases (for example as illustrated in Figs. 20 and 21) the distal end of the cannula portion 8 has a tapered form to aid insertion through the retracted incision. In some cases, especially when the cannula does not have a tapered end, an obturator may be used to aid entry through an incision. The length of the cannula portion 8 can be as short as is necessary to traverse the thickness of a patient's abdominal tissue. The length of the cannula may be selected based on the thickness of the abdominal wall of a patient. The distal end of the cannula portion 8 may therefore be located close to the inner surface of the abdominal tissue which minimises restriction on movement of an instrument within the abdomen - the cannula provides minimal obstruction to this internal movement. Curved instruments may be readily inserted.

In the invention the cannula has a distal end which extends in use distally of the distal ring of the retractor. However, the distal end of the cannula is located adjacent to and as close as possible to the distal ring to provide minimum obstruction to movement of an instrument within the abdomen.

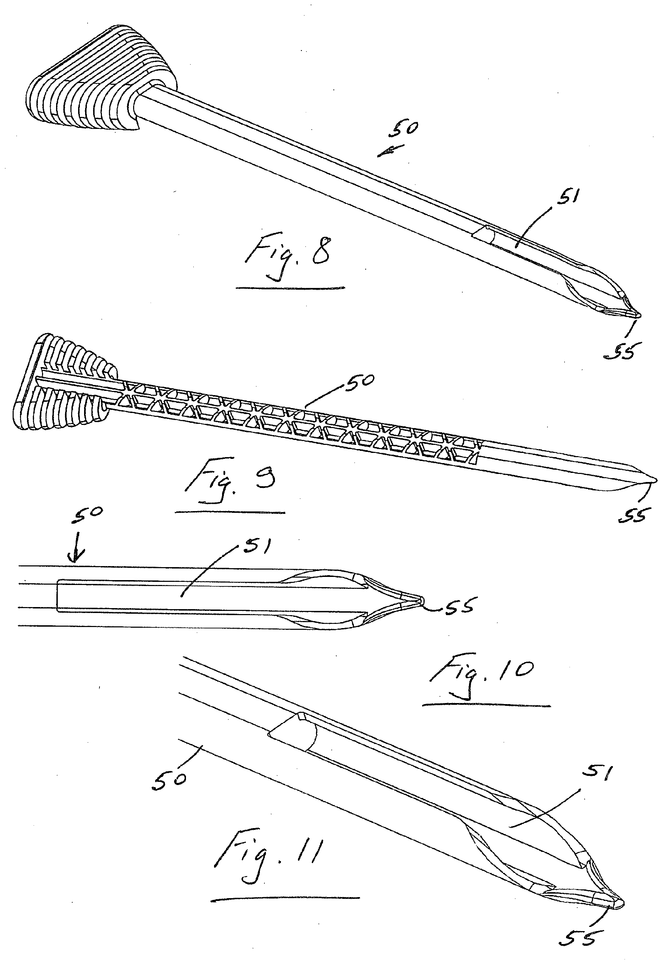

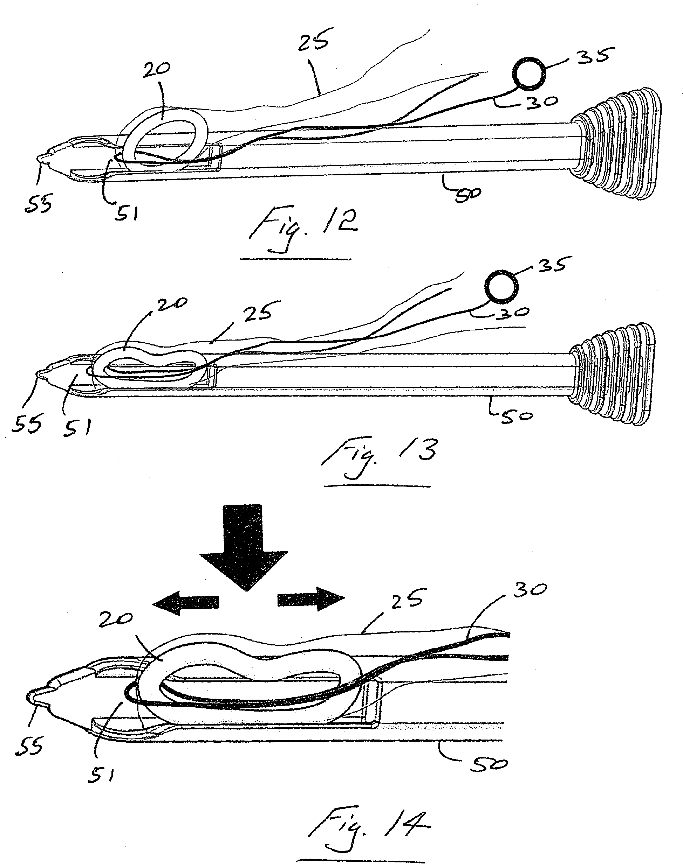

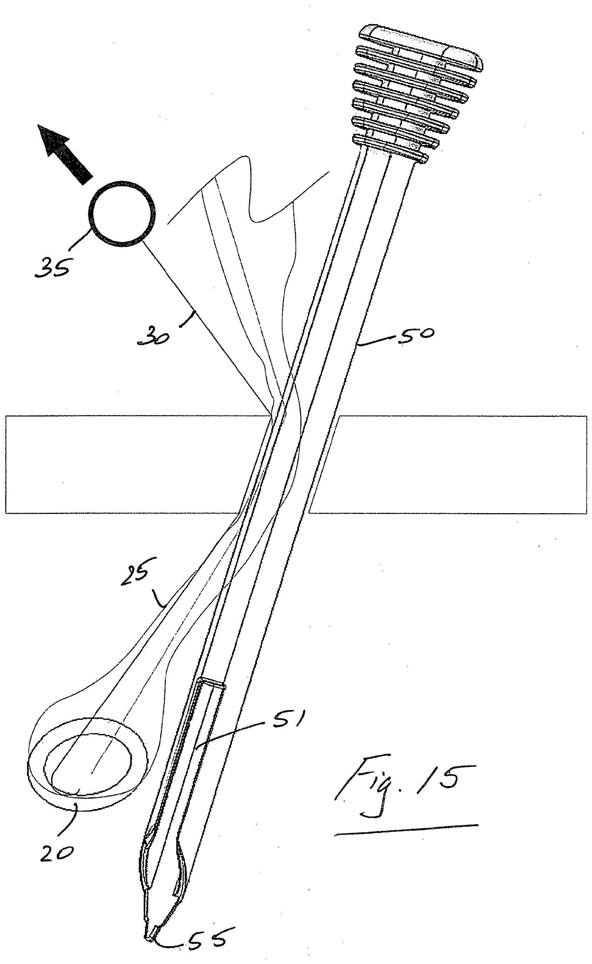

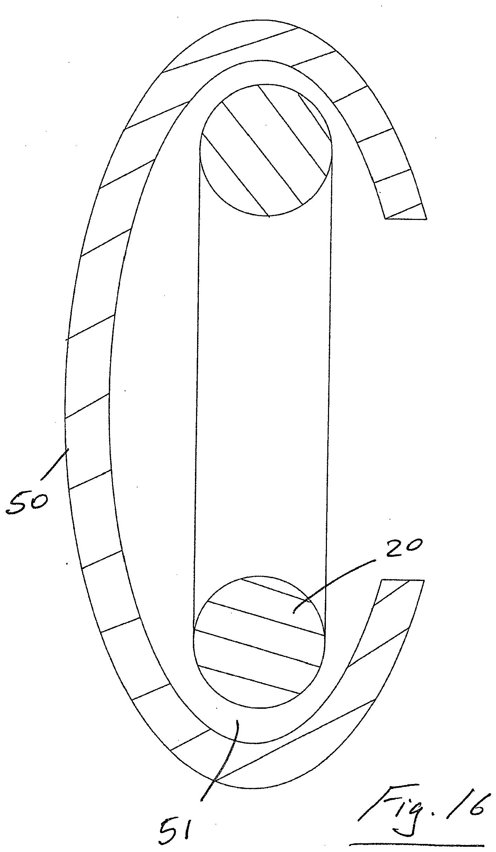

Referring to Figs. 8 to 16 there is illustrated an apparatus 50 according to the invention suitable for inserting a wound retractor device 2 at least partially through a wound opening.

The apparatus 50 comprises a conveying device which is insertable through the incision to convey the wound retractor device 2 through the incision.

An entry/exit opening is provided for the chamber 51. The opening facilitates insertion of the distal ring 20 into the chamber 51 and delivery of the distal ring 20 from within the chamber 51. The sleeve 25 of the wound retractor also extends from the distal ring 20 proximally out of the chamber 51. The device 50 also has a distal tip 55 to aid insertion through an incision.

In use, the wound retractor device 2 is coupled to the conveying device 50 by engaging the distal ring 20 of the wound retractor device 2 in the chamber 51. The device 50 is then passed through the incision with the distal tip 55 as the leading end to assist in opening up the incision. The device 50 is advanced through the wound opening to convey the wound retractor device 2 through the incision. The ring 20 is released by a user pulling on the release ribbon 30. The conveying device 50 may then be withdrawn from the wound opening leaving the wound retractor device 2 in position extending through the wound opening and with the distal ring 20 located distal of the wound opening.

The wound retractor device is coupled to the conveying device 50 by inserting the distal ring 20 into the chamber 51 through the opening. The sleeve portion 25 of the wound retractor device 2 extends proximally out through the opening. The conveying device 50 is then advanced through the wound opening to convey the wound retractor device 2 through the wound opening.

To deploy the retractor, the distal ring 20 and the attached sleeve 25 is pulled from within the chamber 51 through the opening. The conveying device 50 may then be withdrawn from the wound opening leaving the wound retractor device 2 in position extending through the wound opening and with the distal ring 20 distal of the wound opening.

The insertion device 50 used in the invention facilitates entry of a retractor distal ring 20 through the smallest possible incision. Because of the elliptical cross sectional shape the insertion device 50 can be turned as it is being pushed through to open up the incision. The tapered distal tip 55 of the insertion device 50 also facilitates entry and manipulation through the incision. This results in a much smaller incision being required than would usually be required for insertion of a retractor. The distal ring 20 also prevents gas and body fluid (which may be infected) from entering the incision.

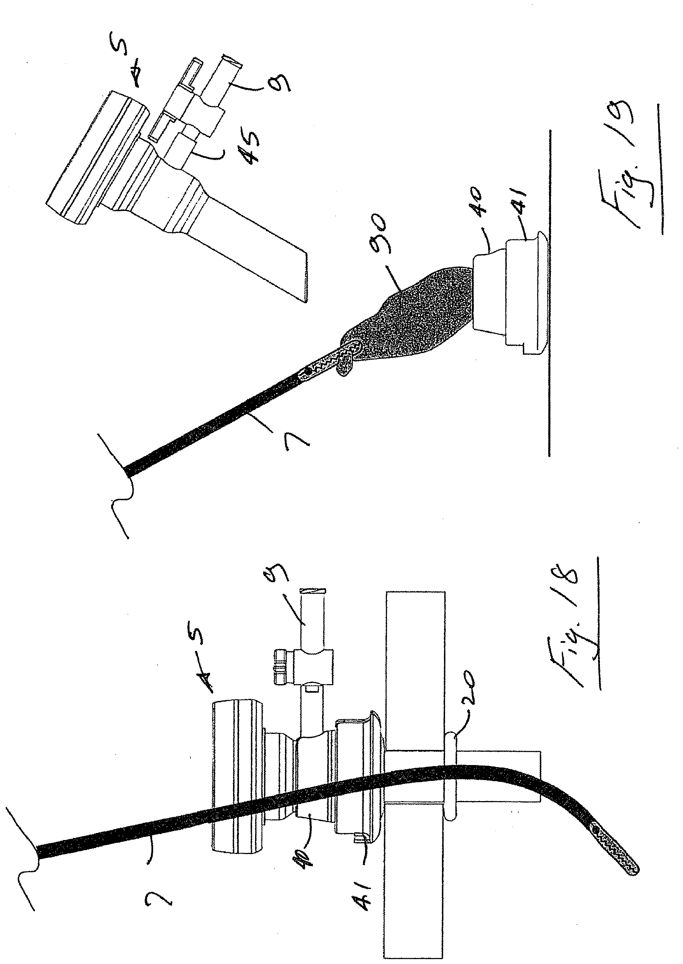

If required, the instrument receiver 5 may be removed from the proximal assembly 3. It may be replaced with another instrument receiver, for example, one which is suitable for insertion of a particular instrument. Indeed, in some cases, as illustrated in Fig. 19 part of the surgical procedure may involve removal of the instrument receiver 5 to facilitate removal of tissue 100 such as a gall bladder or a tissue sample. After removal of the tissue the same or another instrument receiver 5 may be mounted to the proximal assembly.

Referring to Figs. 20 and 21 there is illustrated another instrument access device according to the invention. This device is similar to that described above and like parts are assigned the same reference numerals. In this case the distal end of the cannula 8 is tapered to facilitate insertion through a retracted incision. Only a very short length of the cannula 8 extends beyond the distal ring 20 of the retractor which minimises restriction of the internal movement of instruments inserted through the cannula.

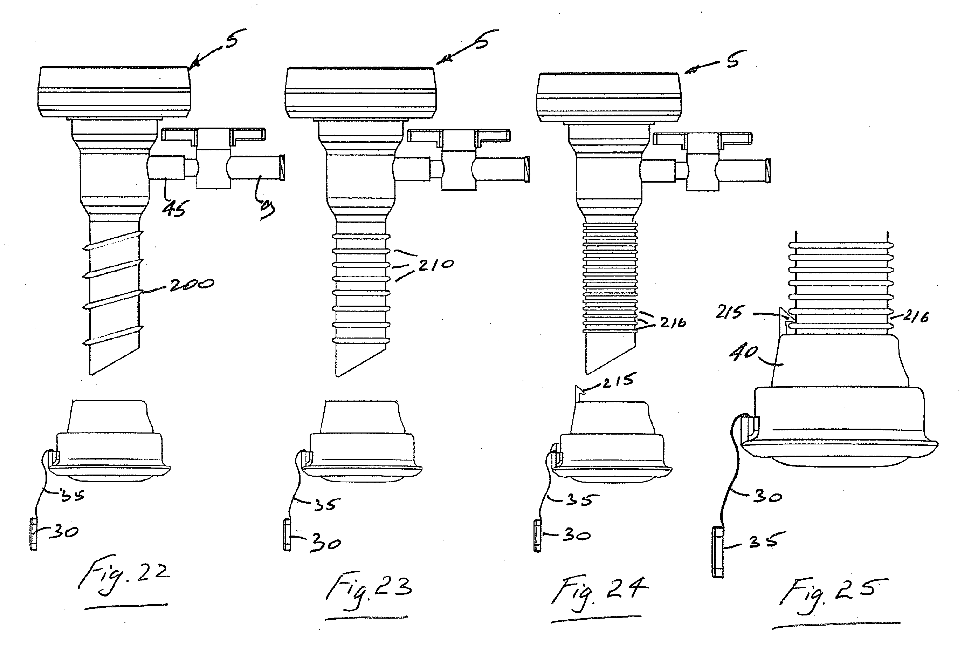

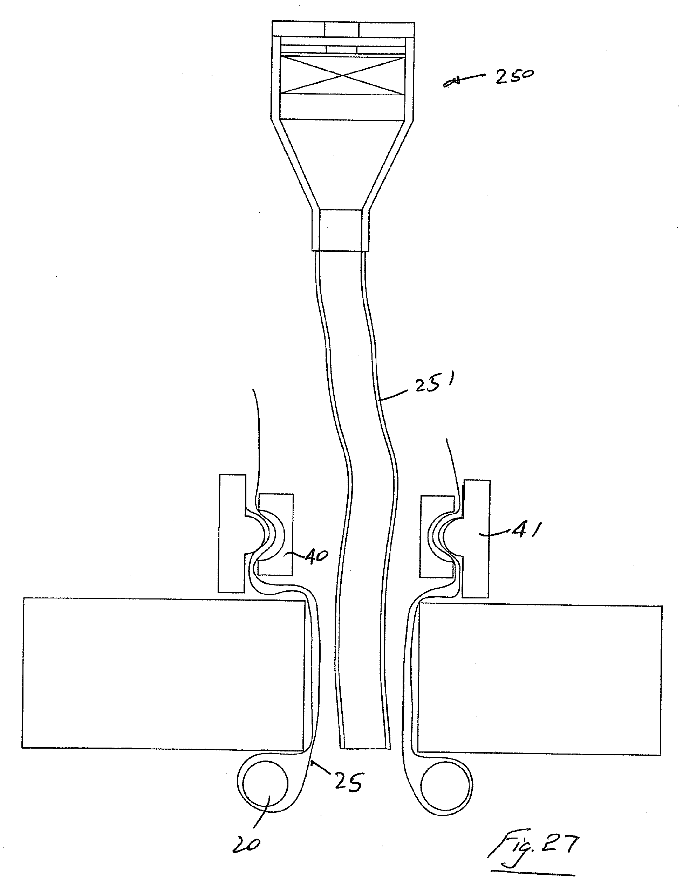

Various alternative instrument access devices are illustrated in Figs. 22 to 28.

Referring to Fig. 22, in this case the cannula portion is provided with projections, for example spiral or screw threads 200 to aid twisting motion as the cannula is inserted. Referring to Fig. 23 the outer surface of the cannula may be provided with ridges/corrugations 210 to provide extra grip.

Referring to Figs. 23 and 24 in this case there is a releasable engagement between the cannula and the proximal ring assembly. The releasable engagement is provided by a pawl 215 on the proximal ring assembly which is engagable in a groove 216 of the cannula somewhat in the manner of a ratchet and pawl mechanism.

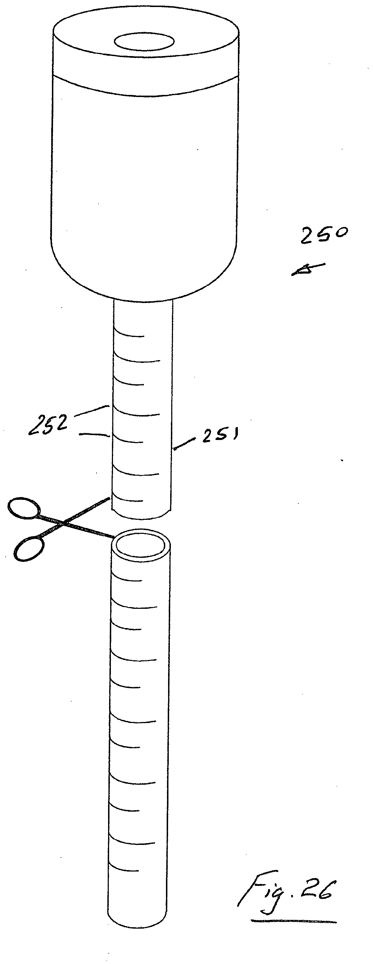

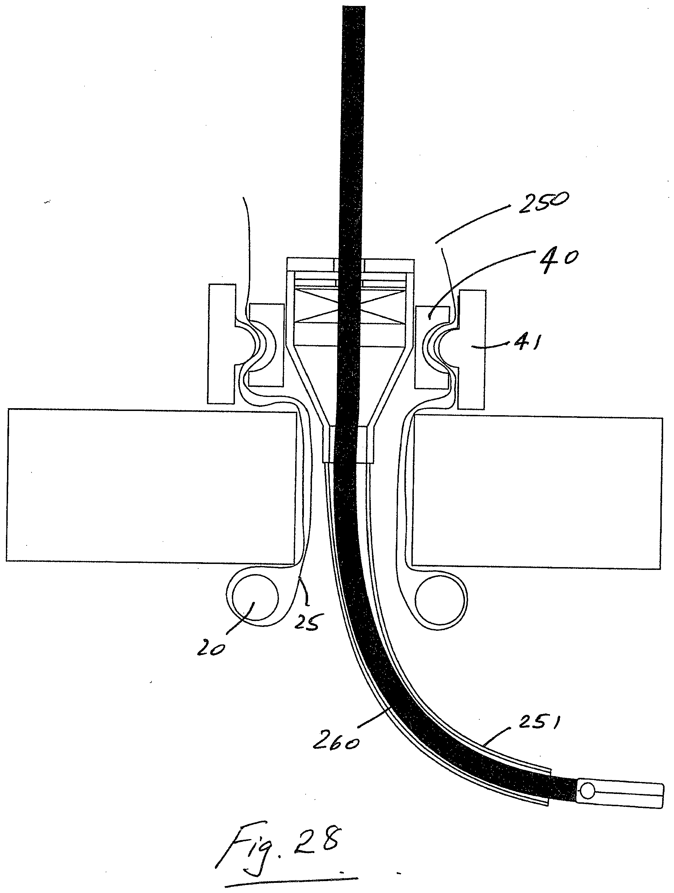

Referring to Figs. 26 to 28, there is illustrated an instrument receiver 250 according to the invention with a cannula portion 251 which can be cut to a desired length to correspond to the thickness of a patient's abdominal tissue. There are indicia in the form of graduation lines 252 which can indicate the length of the cannula portion and thereby act as a guide for cutting to a desired thickness. In this case the cannula portion is also at least partially flexible to further enhance the versatility of the device to accept a range of instruments 260 including a curved instrument 260 as illustrated in Fig. 28. The cannula may be of any material which can be easily cut such as a soft plastic material which would not produce shards on cutting. The cannula portion can conform to any instrument shape.

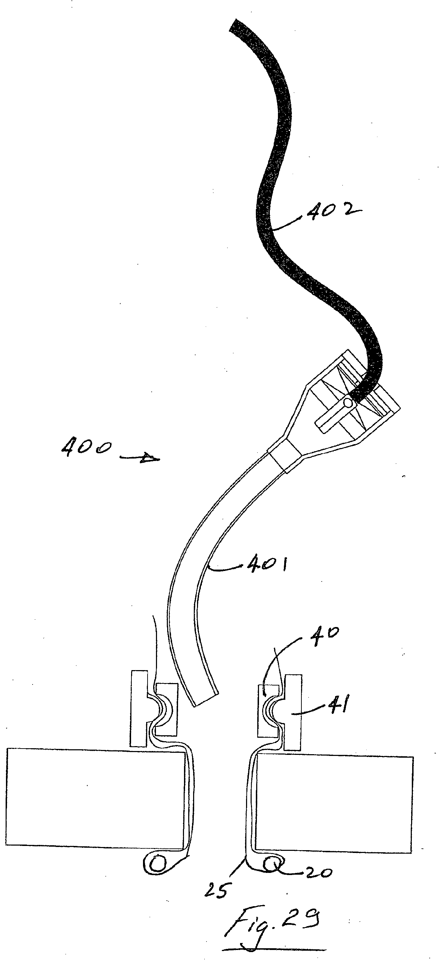

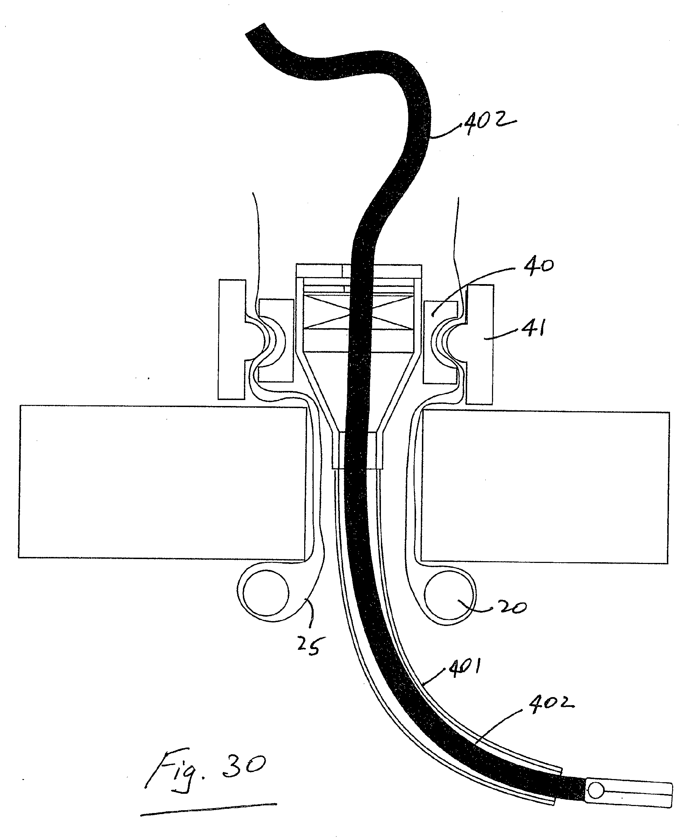

Referring to Figs. 29 and 30, there is illustrated another instrument access device 400 according to the invention which has some features which are similar to those described above. In this case the device 400 has a pre-bent rigid cannula section 401 which is adapted for receiving an instrument with a flexible shaft 402 which can be readily manipulated by the clinician. The cannula may be releasably coupled to the proximal assembly of the retractor. In Fig. 30, the device 400 is illustrated in use in a retracted incision. The flexible instrument is rendered partially rigid a when passed through the cannula.

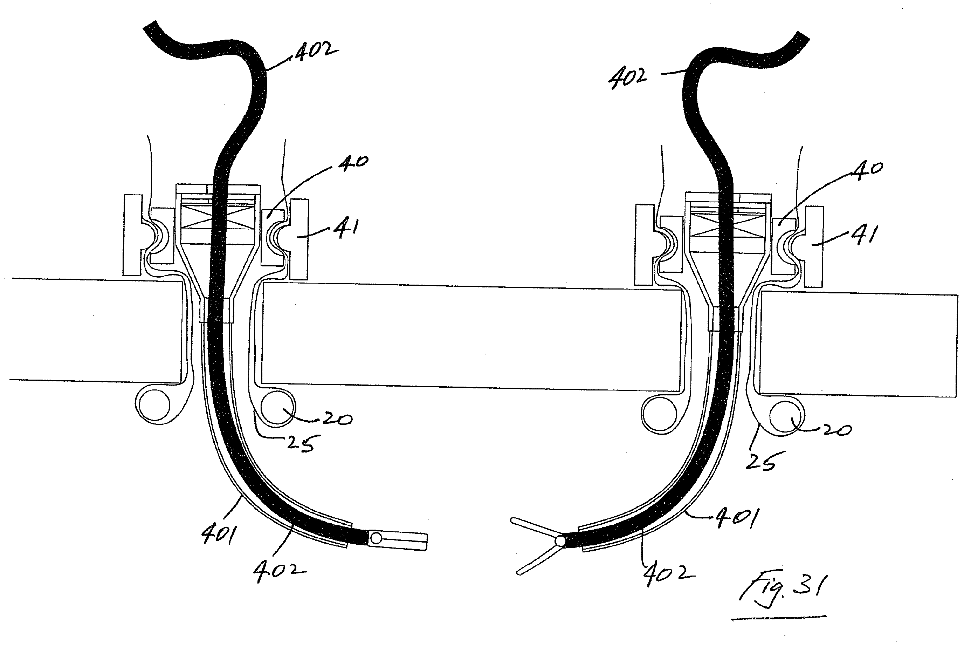

Referring to Fig. 31, an arrangement is illustrated in which two of the devices of Figs. 29 and 30 are in place in two adjacent incision sites. The cannulae and instruments are directed towards one another to facilitate ease of manipulation during surgical procedures.

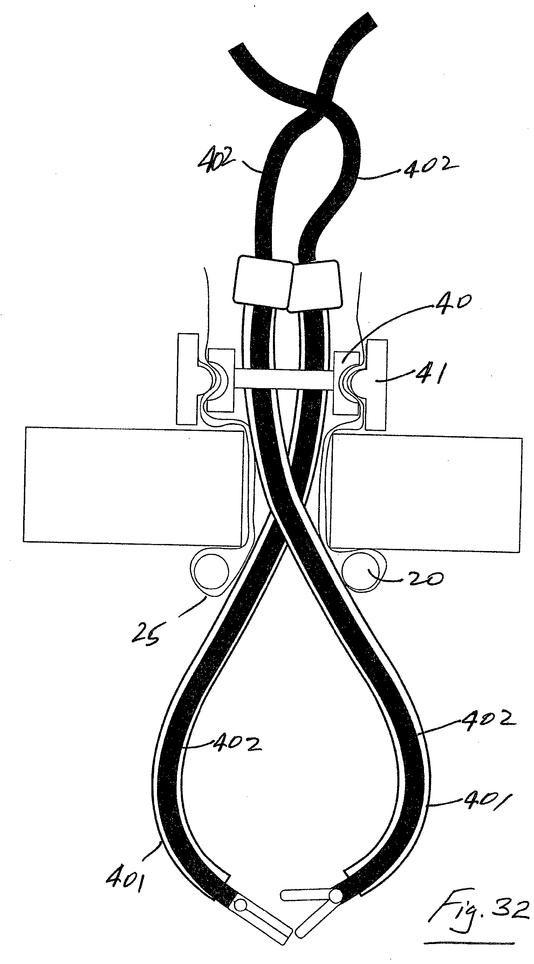

Referring to Fig. 32, in this case two instrument access devices are located in one retracted incision. This avoids the necessity of two separate incisions. There are two independent pre-bent cannula, each with a valve on the proximal end. The flexible instrument is rendered partially rigid a when passed through the cannula.

It will be appreciated that the instrument access devices of the invention may be used in association with robotic systems. The devices may also be used in association with any suitable external attachment such as a dome, a glove, or the like.

The instrument access device of the invention is suitable for use during laparoscopic surgery to facilitate instrument access to an insufflated abdominal cavity while maintaining pneumoperitoneum.

The valve assembly of the instrument receiver 5 in this case comprises a lipseal 10 through which an instrument is insertable and a second seal member 12 having a passageway extending therethrough, through which the instrument is insertable. The device 5 may have a reducer cap which has a further lipseal which is smaller than the lipseal 10. To insert large diameter instruments, the cap is removed. To insert smaller diameter instruments the cap is in place. The second seal member 12 may comprise a duckbill valve through which the instrument passes. The duckbill valve 12 provides sealing engagement with the instrument shaft whilst accommodating lateral movement of the instrument. Alternatively the second valve may comprise a multicusp valve such as a tricuspid valve. In another case the second valve may comprise a foam or gel.

The lipseal valve 10 is located proximally of the duckbill valve 12 so that a double seal is provided to substantially prevent leakage of insufflation gas. The lipseal 10 may be of any suitable material. For example it may be of an elastomeric material, a foam - type material or a gelatinous material. The duckbill valve 12 may be of any suitable material. For example, it may be of a flexible polymeric material.

It will be appreciated that features described with reference to one embodiment of the invention may be utilised with any of the other embodiments.

The invention is not limited to the embodiments hereinbefore described which may be varied in detail.

## Claims

1. An instrument access device (1) comprising:-
a wound protector and retractor (2) comprising a retractor member (25) comprising a distal portion for insertion through an incision made in a patient, a proximal portion for extending from the incision and outside of the patient, and a resilient distal ring member (20) coupled to the retractor member (25);
a proximal assembly (3) comprising an inner proximal ring member (40) and an outer proximal ring member (41) between which the proximal end of the retractor sleeve (25) is located, and an instrument receiver (5, 250) comprising a valve assembly (6) and a cannula portion (8, 251, 401) extending in use through the incision protected by the retractor member (25),
said instrument access device being **characterized in that** the inner proximal ring member (40) comprises a first engagement element (43) and the instrument receiver (5, 250) comprises a second engagement element (45), the first and second engagement elements (43, 45) being releasably coupled for releasably coupling the instrument receiver (5, 250) to the proximal assembly (3).

2. A device as claimed in claim 1 wherein the first engagement element comprises a receiving slot (43).

3. A device as claimed in claim 1 or 2 wherein the cannula portion (8) of the instrument receiver is at least partially flexible.

4. A device as claimed in any of claims 1 to 3 wherein the length of the cannula portion (8) is adjustable.

5. A device as claimed in any of claims 1 to 4 wherein at least portion of the cannula portion (8) is of a material which can be severed to shorten the length of the cannula portion.

6. A device as claimed in any of claims 1 to 5 wherein the valve assembly comprises a first valve (10) and a second valve (12) distal of the first valve (10).

7. A device as claimed in claim 6 wherein the first valve comprises a lipseal valve (10), the second valve (12) may comprise at least two cusps or the second valve may comprise a duckbill valve (12).

8. A device as claimed in claim 7 wherein the lipseal (10) is provided in a lipseal housing and the second valve (12) is provided in a second seal housing.

9. A device as claimed in claim 8 wherein the lipseal housing may be movable relative to the second seal housing, the lipseal housing may comprise a cap for the second seal housing, the lipseal housing may be removable from the second seal housing, the lipseal housing may be releasably connected to the second seal housing, the lipseal housing may be connected to the second seal housing by a hinge connection such as a strap.

10. A device as claimed in claim 8 or 9 wherein the lipseal housing comprises a reducer cap.

11. A device as claimed in any of claims 1 to 10 wherein the wound protector and retractor (2) comprises a distal ring member (20) for location within a wound interior; and a retractor sleeve (25) extending proximally from the distal ring
member (20) to retract laterally the sides of an incision.

12. A device as claimed in claim 11 wherein the retractor member (25) extends at least between the distal ring member (20) and the proximal assembly (3), optionally the retractor member (25) extends in two layers between the distal ring member (20) and the proximal assembly (3).

13. A device as claimed in claim 12 wherein a first end portion of the retractor member (25) is fixed to the proximal assembly (3), optionally the retractor member (25) is movable relative to the distal ring member (20), and/or optionally a second end portion of the retractor member (25) is movable relative to the proximal assembly (3).

14. A device as claimed in claim 12 or 13 wherein the retractor member (25) extends distally from the proximal assembly (3) to the distal ring member (20), is looped around the distal ring member (20), and extends proximally from the distal ring member (20) to the proximal assembly (3).

15. A device as claimed in any of claims 1 to 14 wherein the retractor member (25) extends between an inner part and an outer part of the proximal assembly (3).

## Patentansprüche

1. Instrumentenzugangsvorrichtung (1), die Folgendes umfasst:
eine Wundschutz- und -retraktorvorrichtung (2), umfassend ein Retraktorglied (25), umfassend einen distalen Abschnitt zum Einführen durch eine in einem Patienten gemachte Inzision, einen proximalen Abschnitt zum Erstrecken von der Inzision und außerhalb des Patienten, und ein elastisches distales Ringglied (20), das an das Retraktorglied (25) gekoppelt ist;
eine proximale Anordnung (3), umfassend ein inneres proximales Ringglied (40) und ein äußeres proximales Ringglied (41), zwischen denen das proximale Ende der Retraktorhülse (25) gelegen ist, und eine Instrumentenaufnahme (5, 250), umfassend
eine Ventilanordnung (6) und einen Kanülenabschnitt (8, 251, 401), der sich beim Gebrauch durch die von dem Retraktorglied (25) geschützte Inzision erstreckt,
wobei die Instrumentenzugangsvorrichtung **dadurch gekennzeichnet ist, dass** das innere proximale Ringglied (40) ein erstes Eingriffselement (43) umfasst und die Instrumentenaufnahme (5, 250) ein zweites Eingriffselement (45) umfasst, wobei das erste und das zweite Eingriffselement (43, 45) lösbar gekoppelt sind, um die Instrumentenaufnahme (5, 250) lösbar an die proximale Anordnung (3) zu koppeln.

2. Vorrichtung nach Anspruch 1, wobei das erste Eingriffselement einen Aufnahmeschlitz (43) umfasst.

3. Vorrichtung nach Anspruch 1 oder 2, wobei der Kanülenabschnitt (8) der Instrumentenaufnahme mindestens teilweise biegsam ist.

4. Vorrichtung nach einem der Ansprüche 1 bis 3, wobei die Länge des Kanülenabschnitts (8) verstellbar ist.

5. Vorrichtung nach einem der Ansprüche 1 bis 4, wobei mindestens ein Abschnitt des Kanülenabschnitts (8) aus einem Material ist, das durchtrennt werden kann, um die Länge des Kanülenabschnitts zu kürzen.

6. Vorrichtung nach einem der Ansprüche 1 bis 5, wobei die Ventilanordnung ein erstes Ventil (10) und ein distal von dem ersten Ventil (10) gelegenes zweites Ventil (12) umfasst.

7. Vorrichtung nach Anspruch 6, wobei das erste Ventil ein Lippendichtungsventil (10) umfasst, das zweite Ventil (12) mindestens zwei Segel umfassen kann oder das zweite Ventil ein Entenschnabelventil (12) umfassen kann.

8. Vorrichtung nach Anspruch 7, wobei die Lippendichtung (10) in einem Lippendichtungsgehäuse vorgesehen ist und das zweite Ventil (12) in einem zweiten Dichtungsgehäuse vorgesehen ist.

9. Vorrichtung nach Anspruch 8, wobei das Lippendichtungsgehäuse relativ zu dem zweiten Dichtungsgehäuse beweglich sein kann, wobei das Lippendichtungsgehäuse eine Kappe für das zweite Dichtungsgehäuse umfassen kann, wobei das Lippendichtungsgehäuse von dem zweiten Dichtungsgehäuse abnehmbar sein kann, wobei das Lippendichtungsgehäuse lösbar mit dem zweiten Dichtungsgehäuse verbunden sein kann, wobei das Lippendichtungsgehäuse durch eine Scharnierverbindung, wie etwa einen Gurt, mit dem zweiten Dichtungsgehäuse verbunden sein kann.

10. Vorrichtung nach Anspruch 8 oder 9, wobei das Lippendichtungsgehäuse eine Reduzierkappe umfasst.

11. Vorrichtung nach einem der Ansprüche 1 bis 10, wobei die Wundschutz- und - retraktorvorrichtung (2) ein distales Ringglied (20) zum Positionieren im Inneren einer Wunde und eine Retraktorhülse (25), die sich proximal von dem distalen Ringglied (20) erstreckt, um die Seiten einer Inzision lateral zu spreizen, umfasst.

12. Vorrichtung nach Anspruch 11, wobei sich das Retraktorglied (25) mindestens zwischen dem distalen Ringglied (20) und der proximalen Anordnung (3) erstreckt, wobei sich optional das Retraktorglied (25) in zwei Schichten zwischen dem distalen Ringglied (20) und der proximalen Anordnung (3) erstreckt.

13. Vorrichtung nach Anspruch 12, wobei ein erster Endabschnitt des Retraktorglieds (25) an der proximalen Anordnung (3) fixiert ist, optional das Retraktorglied (25) relativ zu dem distalen Ringglied (20) beweglich ist, und/oder optional ein zweiter Endabschnitt des Retraktorglieds (25) relativ zu der proximalen Anordnung (3) beweglich ist.

14. Vorrichtung nach Anspruch 12 oder 13, wobei sich das Retraktorglied (25) distal von der proximalen Anordnung (3) zu dem distalen Ringglied (20) erstreckt, um das distale Ringglied (20) geschlungen ist und sich proximal von dem distalen Ringglied (20) zu der proximalen Anordnung (3) erstreckt.

15. Vorrichtung nach einem der Ansprüche 1 bis 14, wobei sich das Retraktorglied (25) zwischen einem inneren Teil und einem äußeren Teil der proximalen Anordnung (3) erstreckt.

## Revendications

1. Dispositif d'accès d'instrument (1) comportant :
un dispositif de protection et d'écartement de plaie (2) comportant un élément d'écartement (25) comportant une partie distale à des fins d'insertion au travers d'une incision pratiquée chez un patient, une partie proximale à des fins d'extension depuis l'incision et à l'extérieur du patient, et un élément annulaire distal élastique (20) accouplé à l'élément d'écartement (25) ;
un ensemble proximal (3) comportant un élément annulaire proximal intérieur (40) et un élément annulaire proximal extérieur (41) entre lesquels l'extrémité proximale du manchon d'écartement (25) est située, et un dispositif de réception d'instrument (5, 250) comportant un ensemble soupape (6) et une partie canule (8, 251, 401) s'étendant lors de l'utilisation au travers de l'incision protégée par l'élément d'écartement (25),
ledit dispositif d'accès d'instrument étant **caractérisé en ce que**
l'élément annulaire proximal intérieur (40) comporte un premier élément de mise en prise (43) et le dispositif de réception d'instrument (5, 250) comporte un deuxième élément de mise en prise (45), les premier et deuxième éléments de mise en prise (43, 45) étant accouplés de manière libérable pour accoupler de manière libérable le dispositif de réception d'instrument (5, 250) à l'ensemble proximal (3).

2. Dispositif selon la revendication 1, dans lequel le premier élément de mise en prise comporte une fente de réception (43).

3. Dispositif selon la revendication 1 ou la revendication 2, dans lequel la partie canule (8) du dispositif de réception d'instrument est au moins partiellement flexible.

4. Dispositif selon l'une quelconque des revendications 1 à 3, dans lequel la longueur de la partie canule (8) est ajustable.

5. Dispositif selon l'une quelconque des revendications 1 à 4, dans lequel au moins une partie de la partie canule (8) est réalisée à partir d'un matériau qui peut être coupé pour raccourcir la longueur de la partie canule.

6. Dispositif selon l'une quelconque des revendications 1 à 5, dans lequel l'ensemble soupape comporte une première soupape (10) et une deuxième soupape (12) distale par rapport à la première soupape (10).

7. Dispositif selon la revendication 6, dans lequel la première soupape comporte une soupape à joint à lèvre (10), la deuxième soupape (12) peut comporter au moins deux pointes ou la deuxième soupape peut comporter une soupape en bec de canard (12).

8. Dispositif selon la revendication 7, dans lequel le joint à lèvre (10) est mis en oeuvre dans un logement pour joint à lèvre et la deuxième soupape (12) est mise en oeuvre dans un deuxième logement pour joint.

9. Dispositif selon la revendication 8, dans lequel le logement pour joint à lèvre peut être mobile par rapport au deuxième logement pour joint, le logement pour joint à lèvre peut comporter un bouchon pour le deuxième logement pour joint, le logement pour joint à lèvre peut être en mesure d'être retiré du deuxième logement pour joint, le logement pour joint à lèvre peut être raccordé de manière libérable au deuxième logement pour joint, le logement pour joint à lèvre peut être raccordé au deuxième logement pour joint par un raccord de charnière tel une sangle.

10. Dispositif selon la revendication 8 ou la revendication 9, dans lequel le logement pour joint à lèvre comporte un bouchon réducteur.

11. Dispositif selon l'une quelconque des revendications 1 à 10, dans lequel le dispositif de protection et d'écartement de plaie (2) comporte un élément annulaire distal (20) à des fins de positionnement à l'intérieur de la plaie ; et un manchon d'écartement (25) s'étendant de manière proximale depuis l'élément annulaire distal (20) pour rétracter dans le sens latéral les côtés d'une incision.

12. Dispositif selon la revendication 11, dans lequel l'élément d'écartement (25) s'étend au moins entre l'élément annulaire distal (20) et l'ensemble proximal (3), éventuellement l'élément d'écartement (25) s'étend en deux couches entre l'élément annulaire distal (20) et l'ensemble proximal (3).

13. Dispositif selon la revendication 12, dans lequel une première partie d'extrémité de l'élément d'écartement (25) est fixée sur l'ensemble proximal (3), éventuellement l'élément d'écartement (25) est mobile par rapport à l'élément annulaire distal (20), et/ou éventuellement une deuxième partie d'extrémité de l'élément d'écartement (25) est mobile par rapport à l'ensemble proximal (3).

14. Dispositif selon la revendication 12 ou la revendication 13, dans lequel l'élément d'écartement (25) s'étend de manière distale depuis l'ensemble proximal (3) jusqu'à l'élément annulaire distal (20), est enroulé autour de l'élément annulaire distal (20), et s'étend de manière proximale depuis l'élément annulaire distal (20) jusqu'à l'ensemble proximal (3).

15. Dispositif selon l'une quelconque des revendications 1 à 14, dans lequel l'élément d'écartement (25) s'étend entre une partie intérieure et une partie extérieure de l'ensemble proximal (3).
